(19) Europäisches Patentamt
European Patent Office
Office européen des brevets

(11) Veröffentlichungsnummer: 0 004 898
B1

(12) EUROPÄISCHE PATENTSCHRIFT

(45) Veröffentlichungstag der Patentschrift: 07.04.82

(51) Int. Cl.³: A 61 K 31/565, C 07 J 1/00

(21) Anmeldenummer: 79101000.2

(22) Anmeldetag: 31.03.79

# (54) Feste stabile pharmazeutische Zusammensetzung auf Basis von einem 1,3-Diester des 17-alpha-Ethinyl-7-alpha-methyl-1,3,5(10)-östratrien-1,3,17-beta-triols.

(30) Priorität: 21.04.78 DE 2818164

(43) Veröffentlichungstag der Anmeldung: 31.10.79 Patentblatt 79/22

(45) Bekanntmachung des Hinweises auf die Patenterteilung: 07.04.82 Patentblatt 82/14

(84) Benannte Vertragsstaaten: BE CH DE FR GB IT LU NL SE

(56) Entgegenhaltungen:
FR - A - 1 580 268
US - A - 2 861 086

"Rote Liste" (1980), 75059, 75060, 75111—75113; (1977/78) 75114—75116 J. med-chem- 20(1977), 359—364, DT - A - 1 961 984 Merik Index (1976) S. 3626—3627

(73) Patentinhaber: SCHERING AKTIENGESELLSCHAFT
Berlin und Bergkamen
Müllerstrasse 170/178 Postfach 65 03 11
D-1000 Berlin 65 (DE)

(72) Erfinder: Prezewowsky, Klaus, Dr.
Aarauer Strasse 10
D-1000 Berlin 45 (DE)
Erfinder: Steinbeck, Hermann, Dr.
Hammerstrasse 46
D-1000 Berlin 37 (DE)
Erfinder: Wiechert, Rudolf, Prof. Dr.
Petzower Strasse 8 A
D-1000 Berlin 39 (DE)

EP 0 004 898 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Feste stabile pharmazeutische Zusammensetzung auf Basis von einem 1,3-Diester des 17α-Ethinyl-7α-methyl-1,3,5(10)-östratrien-1,3,17β-triols

Die vorliegende Erfindung betrifft eine feste stabile Zusammensetzung auf Basis von einem 1,3-Diester des 17α-Ethinyl-7α-methyl-1,3,5(10)-östratrien-1,3,17β-triols, dadurch gekennzeichnet, daß sie 0,003 bis 0,05 mg 1,3-Dibenzoesäureester des 17α-Ethinyl-7α-methyl-1,3,5(10)-östratrien-1,3,17β-triols und 0,05 bis 0,5 mg eines Gestagens zusammen mit einem oder mehreren pharmazeutischen Träger(n) oder Verdünnungsmittel(n) pro Dosiseinheit enthält.

In der deutschen Patentschrift 1 593 509 (entsprechend FR - A - 1 580 268) werden 1-Hydroxy-7α-methyl-östradiol-Derivate der allgemeinen Formel

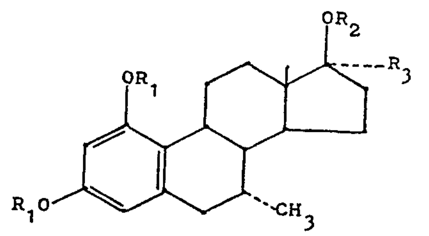

beansprucht, in der

$R_1$ und $R_2$ gleich oder verschieden sind und Wasserstoff, einen niederen Alkyl- oder gesättigten sauerstoffheterocyclischen Rest oder einen physiologisch unbedenklichen Säurerest und

$R_3$ Wasserstoff oder einen gesättigten oder ungesättigten niederen Alkylrest bedeuten.

Die Verbindungen besitzen wertvolle therapeutische Eigenschaften. Sie zeichnen sich insbesondere durch starke östrogene und ovulationshemmende Wirksamkeiten aus. Eine bevorzugte Verbindung der deutschen Patentschrift 17α-Ethinyl-1,3-diacetoxy-7α-methyl-1,3,5(10)-östratrien-17β-ol (I) erwies sich auch bei der klinischen Prüfung auf seine Endometriumswirksamkeit als ein sehr starkes Östrogen. Im Vergleich zu 17α-Ethinyl-östradiol ist Verbindung I etwa 10 mal stärker wirksam.

Bei so hochwirksamen und in Präparaten entsprechend niedrig dosierten Wirkstoffen werden besonders hohe Anforderungen an die Stabilität der Workstoffe gestellt. Es hat sich nun gezeigt, daß bei der trockenen Temperaturbelastung bis 60°C über 2 bis 7 Monate Verbindung I in der gewünschten niedrigen Dosierung eines Präparats instabil ist. Unter der gleichen thermischen Belastung bleibt überraschenderweise der entsprechende 1,3-Dibenzoesäureester stabil.

In der deutschen Patentschrift 1 593 509 (entsprechend FR - A - 1 580 268) wird zwar auch Benzoesäure für die Veresterung von 17α-Ethinyl-7α-methyl-1,3,5(10)-östratrien-1,3,17β-triol vorgeschlagen. Der 1,3-Dibenzoesäureester ist jedoch nicht hergestellt worden, und die überraschenden Eigenschaften des 1,3-Dibenzoesäureesters im Vergleich zum Diessigsaüreester sind infolgendessen auch noch nicht bekannt.

Die Herstellung des neuen Dibenzoesäureesters erfolgt nach an sich bekannten Verfahren. So kann man zum Beispiel von dem in der deutschen patentschrift 1 593 509 (entsprechend FR - A - 1 580 268) beschriebenen 17α-Ethinyl-7α-methyl-1,3,5(10)-östratrien-1,3,17β-triol ausgehen und die freien Hydroxygruppen in 1- und 3-Stellung mit Benzoesäure oder einem Derivat der Benzoesäure in üblicher Weise verestern. Als Derivate kommen vorzugsweise Anhydride und Halogenide, insbesondere das Chlorid, in Frage. Um eine gleichzeitige Veresterung der tertiären 17-Hydroxygruppe zu vermeiden, wird die Veresterung vorzugsweise in Gegenwart einer Base, wie zum Beispiel Pyridin, bei Raumtemperatur durchgeführt. Der neue Dibenzoesäureester wird mit einem oder mehreren pharmazeutischen Träger(n) oder Verdünnungsmittel(n) unter Zusatz eines Gestagens zu festen stabilen Präparaten, wie Tabletten, Dragées oder Kapseln, formuliert werden. Diese Präparate enthalten 0,003 bis 0,05 mg des erfindungsgemäßen Ostrogens und 0,05 bis 0,5 mg eines Gestagens. Die Präparate sollen hauptsächlich zur Kontrazeption bei der Frau angewendet werden.

Beispiel 1

Tabletten können in üblicher Weise aus folgenden Bestandteilen hergestellt werden:

| | |
|---|---|
| 0,010 mg | 17α-Ethinyl-1,3-dibenzoyloxy-7α-methyl-1,3,5(10)-östratrien-17β-ol |
| 0,100 mg | 17α-Ethinyl-17β-hydroxy-18-methyl-4-östren-3-on (Levonorgestrel) |
| 55,290 mg | Lactose |
| 24,000 mg | mikrokristalline Cellulose |
| 0,600 mg | Magnesiumstearat |
| 80,000 mg | Gesamtgewicht der Tablette |

Beispiel 2

Zusammensetzung einer weiteren Tablette:

| | |
|---|---|
| 0,010 mg | 17αEthinyl-1,3-dibenzoyloxy-7α-methyl-1,3,5(10)-östratrien-17β-ol |
| 0,075 mg | 17α-Ethinyl-17β-hydroxy-18-methyl-4,15-östradien-3-on |
| 55,315 mg | Mannit |
| 24,000 mg | mikrokristalline Cellulose |
| 0,600 mg | Magnesiumstearat |
| 80,000 mg | Gesamtgewicht der Tablette |

Beispiel 3

Vergleichende Stabilitätsprüfungen von 17α-Ethinyl-1,3-diacetoxy-7α-methyl-1,3,5(10)-östratrien-17β-ol (I) und 17α-Ethinyl-1,3-dibenzoyloxy-7α-methyl-1,3,5(10)-östratrien-17β-ol (II).

Herstellung von Pulvermischungen

A: Der Wirkstoff wird in Methylenchlorid gelöst, auf Lactose aufgezogen, und das Methylenchlorid wird anschließend verdampft.

B: Mikronisierter Wirkstoff wird mit Lactose verrieben.

Wirkstoffkonzentrationen: 1%; 0,01%; 0,003%.

Lagerung in braunen Standgläsern bei Raumtemperatur (22°C) 40, 50 und 60°C.

Ergebnisse der Gehaltsbestimmung von Diacetat I und Dibenzoat II (siehe Tabelle)

TABELLE

| | 2 Monate | | | | 7 Monate | | | |
|---|---|---|---|---|---|---|---|---|
| Herstellung A | Rt | 40° C | 50° C | 60° C | Rt | 40° C | 50° C | 60° C |
| Diacetate I | | | | | | | | |
| 1 % | 94 % | 94 % | 94 % | 94 % | 95 % | 95 % | 95 % | 95 % |
| 0,01 % | 100 % | 90 % | 87 % | 48 % | 95 % | 83 % | 55 % | 0 % |
| 0,003 % | 99 % | 98 % | 74 % | 34 % | 96 % | 55 % | 0 % | 0 % |
| Dibenzoat II | | | | | | | | |
| 1 % | 95 % | 98 % | 104 % | 100 % | 104 % | 105 % | 100 % | 96 % |
| 0,01 % | 95 % | 104 % | 95 % | 98 % | 102 % | 100 % | 95 % | 105 % |
| 0,003 % | 97 % | 105 % | 104 % | 96 % | 104 % | 105 % | 95f | 89 % |
| Herstellung B | | | | | | | | |
| Diacetat I | | | | | | | | |
| 1 % | 98 % | 96 % | 102 % | 104 % | 98 % | 96 % | 96 % | 94 % |
| 0,01 % | 100 % | 84 % | 73 % | 70 % | 100 % | 77 % | 50 % | 46 % |
| 0,003 % | 99 % | 75 % | 60 % | 29 % | 92 % | 68 % | 23 % | 0 % |
| Dibenzoat II | | | | | | | | |
| 1 % | 99 % | 102 % | 101 % | 105 % | 97 % | 100 % | 103 % | 104 % |
| 0,01 % | 105 % | 102 % | 99 % | 98 % | 103 % | 101 % | 102 % | 95 % |
| 0,003 % | 105 % | 103 % | 102 % | 100 % | 99 % | 96 % | 95 % | 88 % |

## Patentanspruch

Feste stabile pharmazeutische Zusammensetzung auf Basis von einem 1,3-Diester des 17α-Ethinyl-7α-methyl-1,3,5(10)-östratrien-1,3,17β-triols, dadurch gekennzeichnet, daß sie 0,003 bis 0,05 mg 1,3-Dibenzoesäureester des 17α-Ethinyl-7α-methyl-1,3,5(10)-östratrien-1,3,17β-triols und 0,05 bis 0,5 mg eines Gestagens zusammen mit einem oder mehreren pharmazeutischen Träger(n) oder Verdünnungsmittel(n) pro Dosiseinheit enthält.

## Claim

Solid stable pharmaceutical composition based on a 1,3-diester of 17α-ethynyl-7α-methyl-1,3,5(10)-oestratriene-1,3,17β-triol, characterised in that it contains from 0.003 to 0.05 mg of the 1,3-dibenzoic acid ester of 17α-ethynyl-7α-methyl-1,3,5(10)-oestratriene-1,3,17β-triol and from 0.05 to 0.5 mg of a gestagen together with one or more pharmaceutical carrier(s) or diluent(s) per dosage unit.

## Revendication

Composition pharmaceutique solide stable à base d'un diester en 1,3 de l'éthynyl-17α méthyl-7α estratriène-1,3,5(10) triol-1,3,17β, caractérisée en ce qu'elle contient, par unité de prise, de 0,003 à 0,05 mg de l'ester dibenzoique en 1,3 de l'éthynyl-17α-méthyl-7α estratriène-1,3,5(10) triol-1,3,17β et de 0,05 à 0,5 mg d'un gestagène, ainsi qu'un ou plusieurs excipients ou diluants pharmaceutiques.